# FASCICULE DE BREVET EUROPEEN

(11) **EP 1 158 993 B1**
(45) Date de publication et mention de la délivrance du brevet: **29.11.2006**
(21) Numéro de dépôt: 00910946.3
(22) Date de dépôt: 15.03.2000
(51) Int. Cl.: A61K 35/74, A61K 39/155, A61K 39/00, A61K 39/39, A61K 39/385, A61P 37/04, A61P 31/12, A61P 35/00

(54) **FRACTIONS MEMBRANAIRES BACTERIENNES A EFFET ADJUVANT**
BAKTERIENWANDFRAKTIONEN MIT ADJUVANSAKTIVITÄT
BACTERIAL MEMBRANE FRACTIONS WITH ADJUVANT EFFECT

(30) Priorité: 15.03.1999 FR 9903153
(43) Date de publication de la demande: 05.12.2001
(73) Titulaire: PIERRE FABRE MEDICAMENT, 92100 Boulogne (FR)
(72) Inventeur: LIBON, Christine, F-74160 Saint Julien en Genevois (FR); CORVAIA, Nathalie, F-74160 Saint Julien en Genevois (FR); N'GUYEN, Thien, Ngoc, F-74160 Saint Julien en Genevois (FR); BECK, Alain, F-74160 Collonges sous Salève (FR); BONNEFOY, Jean-Yves, F-74350 Le Sappey (FR)
(74) Mandataire: Warcoin, Jacques
(86) Numéro de dépôt international: PCT/FR2000/000622
(87) Numéro de publication internationale: WO 2000/054789

(56) Documents cités:
- WO-A-00/27432
- FR-A- 2 596 064
- FR-A- 2 718 452
- FR-A- 2 726 472
- FR-A- 2 748 476
- FR-A- 2 766 192
- HAEUW J F ET AL: "The recombinant Klebsiella pneumoniae outer membrane protein OmpA has carrier properties for conjugated antigenic peptides" EUROPEAN JOURNAL OF BIOCHEMISTRY, vol. 255, no. 2, 15 juillet 1998 (1998-07-15), pages 446-454, XP002116544
- RAULY I ET AL.: "Carrier properties of a protein derived from outer membrane protein A of Klebsiella pneumoniae" INFECTION AND IMMUNITY, vol. 67, no. 11, novembre 1999 (1999-11), pages 5547-5551, XP002138526

### LISTAGE DE SEQUENCES

<110> PIERRE FABRE MEDICAMENT
<120> UTILISATION DE FRACTIONS MEMBRANAIRES BACTERIENNES A EFFET ADJUVANT, LEURS PROCEDES DE PREPARATION ET COMPOSITION PHARMACEUTIQUE LES CONTENANT.
<130> D17975
<140> FR 99 03513
<141> 1999-03-15
<160> 4
<170> PatentIn Vers. 2.0
<210> 1
<211> 1035
<212> ADN
<213> Klebsiella pneumoniae
<220>
<221> exon
<222> (1)..(1032)
<220>
<221> intron
<222> (1033)..(1035)
<220>
<221> CDS
<222> (1)..(1032)
<400> 1
   atg aaa gca att ttc gta ctg aat gcg gct ccg aaa gat aac acc tgg    48
   Met Lys Ala Ile Phe Val Leu Asn Ala Ala Pro Lys Asp Asn Thr Trp
    1               5                  10                  15
   tat gca ggt ggt aaa ctg ggt tgg tcc cag tat cac gac acc ggt ttc    96
   Tyr Ala Gly Gly Lys Leu Gly Trp Ser Gln Tyr His Asp Thr Gly Phe
               20                  25                  30
   tac ggt aac ggt ttc cag aac aac aac ggt ccg acc cgt aac gat cag    144
   Tyr Gly Asn Gly Phe Gln Asn Asn Asn Gly Pro Thr Arg Asn Asp Gln
           35                  40                  45
   ctt ggt gct ggt gcg ttc ggt ggt tac cag gtt aac ccg tac ctc ggt    192
   Leu Gly Ala Gly Ala Phe Gly Gly Tyr Gln Val Asn Pro Tyr Leu Gly
       50                  55                  60
   ttc gaa atg ggt tat gac tgg ctg ggc cgt atg gca tat aaa ggc agc    240
   Phe Glu Met Gly Tyr Asp Trp Leu Gly Arg Met Ala Tyr Lys Gly Ser
   65                  70                  75                  80
   gtt gac aac ggt gct ttc aaa gct cag ggc gtt cag ctg acc gct aaa    288
   Val Asp Asn Gly Ala Phe Lys Ala Gln Gly Val Gln Leu Thr Ala Lys
                   85                  90                  95
   ctg ggt tac ccg atc act gac gat ctg gac atc tac acc cgt ctg ggc    336
   Leu Gly Tyr Pro Ile Thr Asp Asp Leu Asp Ile Tyr Thr Arg Leu Gly
               100                 105                 110
   ggc atg gtt tgg cgc gct gac tcc aaa ggc aac tac gct tct acc ggc    384
   Gly Met Val Trp Arg Ala Asp Ser Lys Gly Asn Tyr Ala Ser Thr Gly
           115                 120                 125
   gtt tcc cgt agc gaa cac gac act ggc gtt tcc cca gta ttt gct ggc    432
   Val Ser Arg Ser Glu His Asp Thr Gly Val Ser Pro Val Phe Ala Gly
       130                 135                 140
   ggc gta gag tgg gct gtt act cgt gac atc gct acc cgt ctg gaa tac    480
   Gly Val Glu Trp Ala Val Thr Arg Asp Ile Ala Thr Arg Leu Glu Tyr
   145                 150                 155                 160
   cag tgg gtt aac aac atc ggc gac gcg ggc act gtg ggt acc cgt cct    528
   Gln Trp Val Asn Asn Ile Gly Asp Ala Gly Thr Val Gly Thr Arg Pro
                   165                 170                 175
   gat aac ggc atg ctg agc ctg ggc gtt tcc tac cgc ttc ggt cag gaa    576
   Asp Asn Gly Met Leu Ser Leu Gly Val Ser Tyr Arg Phe Gly Gln Glu
               180                 185                 190
   gat gct gca ccg gtt gtt gct ccg gct ccg gct ccg gct ccg gaa gtg    624
   Asp Ala Ala Pro Val Val Ala Pro Ala Pro Ala Pro Ala Pro Glu Val
           195                 200                 205
   gct acc aag cac ttc acc ctg aag tct gac gtt ctg ttc aac ttc aac    672
   Ala Thr Lys His Phe Thr Leu Lys Ser Asp Val Leu Phe Asn Phe Asn
       210                 215                 220
   aaa gct acc ctg aaa ccg gaa ggt cag cag gct ctg gat cag ctg tac    720
   Lys Ala Thr Leu Lys Pro Glu Gly Gln Gln Ala Leu Asp Gln Leu Tyr
   225                 230                 235                 240
   act cag ctg agc aac atg gat ccg aaa gac ggt tcc gct gtt gtt ctg    768
   Thr Gln Leu Ser Asn Met Asp Pro Lys Asp Gly Ser Ala Val Val Leu
                   245                 250                 255
   ggc tac acc gac cgc atc ggt tcc gaa gct tac aac cag cag ctg tct    816
   Gly Tyr Thr Asp Arg Ile Gly Ser Glu Ala Tyr Asn Gln Gln Leu Ser
               260                 265                 270
   gag aaa cgt gct cag tcc gtt gtt gac tac ctg gtt gct aaa ggc atc    864
   Glu Lys Arg Ala Gln Ser Val Val Asp Tyr Leu Val Ala Lys Gly Ile
           275                 280                 285
   ccg gct ggc aaa atc tcc gct cgc ggc atg ggt gaa tcc aac ccg gtt    912
   Pro Ala Gly Lys Ile Ser Ala Arg Gly Met Gly Glu Ser Asn Pro Val
       290                 295                 300
   act ggc aac acc tgt gac aac gtg aaa gct cgc gct gcc ctg atc gat    960
   Thr Gly Asn Thr Cys Asp Asn Val Lys Ala Arg Ala Ala Leu Ile Asp
   305                 310                 315                 320
   tgc ctg gct ccg gat cgt cgt gta gag atc gaa gtt aaa ggc tac aaa    1008
   Cys Leu Ala Pro Asp Arg Arg Val Glu Ile Glu Val Lys Gly Tyr Lys
                   325                 330                 335
   gaa gtt gta act cag ccg gcg ggt taa                                1035
   Glu Val Val Thr Gln Pro Ala Gly
               340
<210> 2
<211> 344
<212> PRT
<213> Klebsiella pneumoniae
<400> 2
   Met Lys Ala Ile Phe Val Leu Asn Ala Ala Pro Lys Asp Asn Thr Trp
    1               5                  10                  15
   Tyr Ala Gly Gly Lys Leu Gly Trp Ser Gln Tyr His Asp Thr Gly Phe
               20                  25                  30
   Tyr Gly Asn Gly Phe Gln Asn Asn Asn Gly Pro Thr Arg Asn Asp Gln
           35                  40                  45
   Leu Gly Ala Gly Ala Phe Gly Gly Tyr Gln Val Asn Pro Tyr Leu Gly
       50                  55                  60
   Phe Glu Met Gly Tyr Asp Trp Leu Gly Arg Met Ala Tyr Lys Gly Ser
   65                  70                  75                  80
   Val Asp Asn Gly Ala Phe Lys Ala Gln Gly Val Gln Leu Thr Ala Lys
                   85                  90                  95
   Leu Gly Tyr Pro Ile Thr Asp Asp Leu Asp Ile Tyr Thr Arg Leu Gly
               100                 105                 110
   Gly Met Val Trp Arg Ala Asp Ser Lys Gly Asn Tyr Ala Ser Thr Gly
           115                 120                 125
   Val Ser Arg Ser Glu His Asp Thr Gly Val Ser Pro Val Phe Ala Gly
       130                 135                 140
   Gly Val Glu Trp Ala Val Thr Arg Asp Ile Ala Thr Arg Leu Glu Tyr
   145                 150                 155                 160
   Gln Trp Val Asn Asn Ile Gly Asp Ala Gly Thr Val Gly Thr Arg Pro
                   165                 170                 175
   Asp Asn Gly Met Leu Ser Leu Gly Val Ser Tyr Arg Phe Gly Gln Glu
               180                 185                 190
   Asp Ala Ala Pro Val Val Ala Pro Ala Pro Ala Pro Ala Pro Glu Val
           195                 200                 205
   Ala Thr Lys His Phe Thr Leu Lys Ser Asp Val Leu Phe Asn Phe Asn
       210                 215                 220
   Lys Ala Thr Leu Lys Pro Glu Gly Gln Gln Ala Leu Asp Gln Leu Tyr
   225                 230                 235                 240
   Thr Gln Leu Ser Asn Met Asp Pro Lys Asp Gly Ser Ala Val Val Leu
                   245                 250                 255
   Gly Tyr Thr Asp Arg Ile Gly Ser Glu Ala Tyr Asn Gln Gln Leu Ser
               260                 265                 270
   Glu Lys Arg Ala Gln Ser Val Val Asp Tyr Leu Val Ala Lys Gly Ile
           275                 280                 285
   Pro Ala Gly Lys Ile Ser Ala Arg Gly Met Gly Glu Ser Asn Pro Val
       290                 295                 300
   Thr Gly Asn Thr Cys Asp Asn Val Lys Ala Arg Ala Ala Leu Ile Asp
   305                 310                 315                 320
   Cys Leu Ala Pro Asp Arg Arg Val Glu Ile Glu Val Lys Gly Tyr Lys
                   325                 330                 335
   Glu Val Val Thr Gln Pro Ala Gly
               340
<210> 3
<211> 303
<212> ADN
<213> Virus respiratoire syncytial, G2Na
<220>
<221> CDS
<222> (1)..(303)
<400> 3
   acc gtg aaa acc aaa aac acc acg acc acc cag acc cag ccg agc aaa    48
   Thr Val Lys Thr Lys Asn Thr Thr Thr Thr Gln Thr Gln Pro Ser Lys
     1               5                  10                  15
   ccg acc acc aaa cag cgt cag aac aaa ccg ccg aac aaa ccg aac aac    96
   Pro Thr Thr Lys Gln Arg Gln Asn Lys Pro Pro Asn Lys Pro Asn Asn
                20                  25                  30
   gat ttc cat ttc gaa gtg ttc aac ttc gtg ccg tgc agc atc tgc agc   144
   Asp Phe His Phe Glu Val Phe Asn Phe Val Pro Cys Ser Ile Cys Ser
            35                  40                  45
   aac aac ccg acc tgc tgg gcg atc tgc aaa cgt atc ccg aac aaa aaa   192
   Asn Asn Pro Thr Cys Trp Ala Ile Cys Lys Arg Ile Pro Asn Lys Lys
        50                  55                  60
   ccg ggc aaa aaa acc acg acc aaa ccg acc aaa aaa ccg acc ttc aaa   240
   Pro Gly Lys Lys Thr Thr Thr Lys Pro Thr Lys Lys Pro Thr Phe Lys
    65                  70                  75                  80
   acc acc aaa aaa gat cat aaa ccg cag acc acc aaa ccg aaa gaa gtg   288
   Thr Thr Lys Lys Asp His Lys Pro Gln Thr Thr Lys Pro Lys Glu Val
                    85                  90                  95
   ccg acc acc aaa ccg                                               303
   Pro Thr Thr Lys Pro
               100
<210> 4
<211> 101
<212> PRT
<213> Virus respiratoire syncytial, G2Na
<400> 4
   Thr Val Lys Thr Lys Asn Thr Thr Thr Thr Gln Thr Gln Pro Ser Lys
     1               5                  10                  15
   Pro Thr Thr Lys Gln Arg Gln Asn Lys Pro Pro Asn Lys Pro Asn Asn
                20                  25                  30
   Asp Phe His Phe Glu Val Phe Asn Phe Val Pro Cys Ser Ile Cys Ser
            35                  40                  45
   Asn Asn Pro Thr Cys Trp Ala Ile Cys Lys Arg Ile Pro Asn Lys Lys
        50                  55                  60
   Pro Gly Lys Lys Thr Thr Thr Lys Pro Thr Lys Lys Pro Thr Phe Lys
    65                  70                  75                  80
   Thr Thr Lys Lys Asp His Lys Pro Gln Thr Thr Lys Pro Lys Glu Val
                    85                  90                  95
   Pro Thr Thr Lys Pro
               100

## Description

La présente invention concerne l'utilisation d'une fraction membranaire de bactéries à gram négatif Klebsiella *pneumoniae,* associée à un antigène ou haptène pour la préparation d'une composition pharmaceutique destinée à orienter la réponse immunitaire vers une réponse de type Th1 et/ou de type mixte Th1/Th2 dirigée contre ledit antigène ou haptène. L'invention comprend en outre les compositions pharmaceutiques les contenant et leurs applications à la prévention et au traitement de maladies infectieuses, en particulier les infections à paramyxovirus telles que le VRS, et les cancers, notamment ceux dont les tumeurs sont associées à des antigènes tumoraux.

La vaccination est un moyen efficace de prévenir ou de réduire notamment les infections. Le succès des campagnes de vaccination dans ce domaine a permis d'étendre le concept des vaccins aux domaines des maladies auto-immunes, du cancer et de la fertilité. En revanche, les antigènes vaccinants ne sont pas toujours capables d'induire seuls une réponse anticorps rapide et soutenue, ce qui requiert la présence d'adjuvants, c'est-à-dire de composés qui les aident (du latin *adjuvare* : aider) à induire de telles réponses.

Les adjuvants constituent un groupe de composés variés quant à leur structure et leur origine. Ainsi l'on trouve, entre autres, dans cette catégorie aussi bien des émulsions eau dans huile (adjuvant incomplet de Freund) ou huile dans eau, des composés d'origine bactérienne comme des dérivés du lipopolysaccharide des bactéries à Gram négatif, que des sels d'aluminium. A l'heure actuelle, seuls les sels d'aluminium sont employés chez l'homme comme adjuvant de préparations vaccinales.

La mise en place d'une réponse anticorps dirigée contre un antigène nécessite une série d'événements complexes. Elle met en jeu des cellules présentant !'antigène, des lymphocytes T régulateurs (Th pour T "helper") et des lymphocytes B producteurs d'anticorps. Deux types de lymphocytes Th peuvent être distingués selon le profil de cytokines produites : lymphocytes Th de type 1 produisant de l'IFN-γ et de l'IL-2 et favorisant la formation d'IgG2a chez la souris, et lymphocytes Th de type 2 produisant de l'IL-4, IL-5 et IL-10 avec formation d'IgG1 chez la souris (Mosmann, T.R. and Sad S. Immunol. Today 1996, 17:138). Par ailleurs, il a été montré que, pour un même antigène donné, c'est l'adjuvant qui oriente vers l'isotype majoritaire lors de la réponse anticorps (Toellner K.-M. et al. J. Exp. Med. 1998, 187:1193). Ainsi, il est connu que les sels d'aluminium, comme l'Alhydrogel, induisent chez la souris une réponse essentiellement de type Th2 et favorisent la formation d'IgG1 voire d'IgE (Allison A.C. In Vaccine design - The role of cytokine networks Vol. 293, 1-9 Plenum Press 1997), ce qui peut poser des problèmes chez des sujets à terrain allergique. De plus, en fonction de la cible thérapeutique visée, une réponse de type Th1 ou mixte (Th1/Th2) peut être désirée.

Ainsi, il existe aujourd'hui un besoin de disposer de nouveaux adjuvants capables d'induire une réponse immune de type Th1 ou mixte (Th1/Th2), de préférence une réponse mixte Th1/Th2 pour laquelle la réponse Th1 est voisine ou supérieure à la réponse Th2.

De manière surprenante, les auteurs de la présente invention ont mis en évidence des propriétés particulières de la fraction membranaire d'une bactérie à gram négatif Klebsiella *pneumoniae* (dénommée FMKp), notamment des fractions membranaires obtenues par les procédés tels que décrits ci-après dans les exemples. Les auteurs ont en effet découvert que ladite fraction membranaire FMKp associée à un antigène avait non seulement la capacité d'augmenter la réponse anticorps dirigée contre ledit antigène mais également de réorienter la réponse cytokinique vers un profil Th1/Th2, correspondant ainsi à l'activité adjuvante particulière recherchée, et ce quel que soit le mode d'administration desdites fractions membranaires.

Ainsi, la présente invention a pour objet l'utilisation d'une fraction membranaire de Klebsiella *pneumoniae,* associée à un antigène ou haptène pour orienter la réponse immunitaire vers une réponse de type Th1 et/ou de type mixte Th1/Th2 dirigée contre ledit antigène ou haptène, ou pour la préparation d'une composition pharmaceutique destinée à orienter la réponse immunitaire vers une réponse de type Th1 et/ou de type mixte Th1/Th2 dirigée contre ledit antigène ou haptène caractérisée en ce que la fraction membranaire est préparée par l'un des deux procédés tels que ci-aprés décrits.

Par orientation de la réponse immune vers une réponse Th1 et/ou de type mixte Th1/Th2, on préfère notamment une orientation de la réponse immune qui favorise l'induction d'une réponse Th1 par rapport à la réponse Th1/Th2 obtenue avec l'adjuvant alum.

Par orientation de la réponse immune vers une réponse Th1 et/ou de type mixte Th1/Th2, on préfère plus particulièrement une orientation de la réponse immune qui augmente le titre en anticorps IgG2a dirigés contre l'antigène associé par un facteur d'au moins 10, de préférence d'au moins 25, 50 et 100 par rapport au titre en IcG2a obtenu avec l'adjuvant alum.

De manière particulièrement préférée, la réponse immune est orientée vers une réponse Th1 et/ou mixte de type Th1/Th2 dans laquelle la réponse Th1 est voisine ou supérieure à la réponse Th2. Par "voisine", on entendra désigner une réponse qui, exprimée en titre d'anticorps IgG2a dirigés contre l'antigène associé, est au moins égale à 0.5 fois, de préférence au moins égale à 0,75 fois, le titre en anticorps IaG1 dirigé contre ledit antigène, avec un titre d'anticorps IgG dirigé contre l'antigène associé voisin ou supérieur au titre d'anticorps IgG dirigé contre l'antigène associé obtenu avec l'adjuvant alum ou de Freund.

L'invention concerne également l'utilisation selon l'invention, caractérisée en ce que la fraction membranaire comprend au moins des fractions membranaires de deux souches différentes de bactéries.

L'invention est donc relative à une utilisation selon l'invention, caractérisée en ce que la fraction membranaire est préparée par un procédé comprenant les étapes suivantes :
a) la culture desdites bactéries dans un milieu de culture permettant leur croissance suivie d'une centrifugation de ladite culture ;
b) le cas échéant, désactivation des enzymes lytiques du culot de bactéries obtenu à l'étape a), puis centrifugation de la suspension obtenue ;
c) extraction et élimination des protéines non membranaires et des acides nucléiques du culot obtenu à l'étape a) ou b) par au moins un cycle de lavage du culot dans une solution d'extraction ;
d) digestion du culot de membranes obtenu à l'étape c) en présence d'enzymes protéolytiques suivie d'une centrifugation ;
e) au moins un cycle de lavage du culot obtenu à l'étape d) dans une solution physiologique et/ou dans de l'eau distillée ; et
f) ultrasonication du culot obtenu à l'étape e).

L'étape b) de désactivation des enzymes lytiques du culot de bactéries obtenu à l'étape a) peut être réalisée par toutes méthodes connues de désactivation d'enzymes, telles qu'en particulier par chauffage du culot bactérien remis en suspension à une température de préférence voisine de 100°C, ou par addition d'inhibiteur de l'activité de ces enzymes.

L'étape c) d'extraction et d'élimination des protéines non membranaires et des acides nucléiques du culot obtenu à l'étape a) ou b) peut être réalisée par exemple par au moins un cycle de lavage du culot dans une solution d'extraction correspondant à l'addition d'une solution hypertonique (solution d'extraction), de préférence une solution saline de molarité voisine de 1 M, suivie après un temps de contact suffisant pour l'effet désiré d'une centrifugation de la suspension obtenue et de l'élimination du surnageant obtenu après ladite centrifugation, ce cycle de lavage pouvant être reproduit plusieurs fois.

L'étape d) de digestion du culot de membranes obtenu à l'étape c) peut être réalisée en présence d'une solution d'enzymes protéolytiques telles que par exemple la trypsine, la chymotrypsine ou toute enzyme à activité protéolytique connue, les conditions de la réaction, pH de la solution, température et durée de la réaction, étant de préférence ajustées aux conditions optimales pour l'activité de ou des enzymes choisies, suivie d'une centrifugation, ce cycle de digestion pouvant être reproduit plusieurs fois avec la même enzyme, la même combinaison d'enzymes ou avec une enzyme différente pour chaque cycle de digestion effectué.

L'étape e) de lavage du culot obtenu à l'étape d) est réalisée par reprise du culot dans une solution physiologique ou dans de l'eau distillée suivie, après un temps de contact suffisant, d'une centrifugation, ce cycle de lavage pouvant être reproduit plusieurs fois.

Enfin, l'étape f) d'ultrasonication, du culot a pour objectif en particulier de désintégrer et d'homogénéiser la fraction membranaire obtenue en fin d'étape e). Les conditions d'ultrasonication (durée et puissance) seront déterminées par l'homme de l'art en fonction par exemple de la quantité de fraction membranaire à traiter.

L'invention est aussi relative à une utilisation selon l'invention, caractérisée en ce que la fraction membranaire est préparée par un procédé comprenant les étapes suivantes :
a) la culture desdites bactéries dans un milieu de culture permettant leur croissance suivie le cas échéant d'une centrifugation ;
b) la congélation du milieu de culture ou du culot obtenu à l'étape a) suivie d'une décongélation et du séchage des cellules ;
c) élimination au moyen d'une DNase des acides nucléiques des cellules sèches obtenues à l'étape b) remises en suspension ;
d) broyage des cellules obtenues à l'étape c) et clarification de la suspension obtenue ;
e) précipitation en milieu acide de la suspension obtenue à l'étape d) et élimination du culot ;
f) neutralisation du surnageant obtenu à l'étape e) contenant la suspension membranaire, suivie d'une dialyse et d'une concentration de la suspension membranaire ; et
g) stérilisation de la suspension membranaire concentrée obtenue à l'étape f).

Les conditions de décongélation à l'étape b) du procédé ci-dessous seront bien entendu déterminées par l'homme de l'art en fonction de la quantité de culot initiale à traiter, de préférence réalisée à 4 °C pendant au moins 48 heures pour l'équivalent de 1 Kg de cellules sèches.

A l'étape c), l'élimination des acides nucléiques est réalisée par exemple par l'addition d'une DNase, à une concentration finale de 5 mg/ml d'une suspension de cellules à une concentration équivalente à 5 % de cellules sèches.

Le broyage des cellules obtenues à l'étape c) peut être réalisé au moyen de tout système ou appareillage connu par l'homme de l'art pour le broyage de cellules tel que les presses ou de préférence tel que le broyage en boucle de Manton Gaulinet pendant 30 minutes.

La clarification de la suspension obtenue après broyage pourra être réalisée au moyen de tout système ou appareillage connu par l'homme de l'art pour la clarification de broyats cellulaires bactériens tel que le système Sharpless.

L'étape e) de précipitation en milieu acide de la suspension obtenue à l'étape d) peut être réalisée par exemple avec l'acide acétique. La précipitation est suivie par l'élimination du culot au moyen d'un système de type Sharpless et par la récupération du surnageant.

L'étape f) consiste en une étape dans laquelle le surnageant, obtenu après précipitation en milieu acide, est neutralisé, dilué, dialysé puis concentré.

Enfin, la dernière étape consiste en une étape de stérilisation du concentré de fraction membranaire obtenu à l'étape précédente comme par exemple par chauffage à 121 °C pendant environ 35 minutes.

L'invention concerne de manière particulière l'utilisation selon l'invention, caractérisée en ce que ledit antigène ou haptène est choisi parmi les antigènes ou haptènes spécifiques d'un agent infectieux, tel qu'un virus, une bactérie, un champignon ou un parasite, ou parmi les antigènes associés à des cellules tumorales.

Selon l'invention, lesdits antigènes ou haptènes sont de préférence choisis parmi les peptides, les lipopeptides, les polysaccharides, les oligosaccharides, les acides nucléiques ou les lipides.

Bien entendu, ledit antigène ou haptène, lorsqu'il est de nature peptidique pourra être obtenu par synthèse chimique ou recombinante.

Les méthodes de préparation de peptides recombinants sont aujourd'hui bien connues de l'homme de l'art et ne seront pas développées dans la présente description. Parmi les cellules utilisables pour la production de ces peptides recombinants, il faut citer bien entendu les cellules bactériennes (Olins P.O. et Lee S.C., 1993, Recent advances in heterologous gene expression in E. coli. Curr. Op. Biotechnology 4:520-525), mais également les cellules de levure (Buckholz R.G., 1993, Yeast Systems for the Expression of Heterologous Gene Products. Curr. Op. Biotechnology 4:538-542), de même que les cellules animales, en particulier les cultures de cellules de mammifères (Edwards C.P. et Aruffo A., 1993, Current applications of COS cell based transient expression systems. Curr. Op. Biotechnology 4, 558-563) mais également les cellules d'insectes dans lesquelles on peut utiliser des procédés mettant en oeuvre par exemple des baculovirus (Luckow V.A., 1993, Baculovirus systems for the expression of human gene products. Curr. Op. Biotechnology 4, 564-572).

Dans un mode préféré de réalisation, l'invention comprend l'utilisation selon l'invention, caractérisée en ce que ledit antigène ou haptène est couplé de manière covalente à un peptide support pour former un complexe capable de se lier spécifiquement à la sérumalbumine de mammifère, de préférence ledit peptide support est un fragment peptidique issu de la protéine G du streptocoque, notamment le fragment C-terminal dénommé BB.

Bien entendu, ledit complexe pourra être préparé par recombinaison génétique.

Il est décrit ici que complexe chimérique ou hybride peut être produit par des techniques d'ADN recombinant par insertion ou addition à une séquence d'ADN codant pour ledit fragment peptidique de la protéine G de streptococcus d'une séquence codant pour ledit antigène ou haptène de nature protéique.

Les procédés de synthèse des molécules hybrides englobent les méthodes utilisées en génie génétique pour construire des polynucléotides hybrides codant pour les séquences polypeptidiques recherchées. On pourra, par exemple, se référer avantageusement à la technique d'obtention de gènes codant pour des protéines de fusion décrite par D.V. Goeddel (Gene expression technology, Methods in Enzymology, vol. 185, 3-187, 1990).

L'invention a également pour objet l'utilisation selon l'invention, caractérisée en ce que la composition pharmaceutique comprend en outre un agent permettant de véhiculer ladite fraction membranaire associée audit antigène, haptène ou complexe sous une forme permettant d'améliorer sa stabilité et/ou son immunogénicité, telle que sous la forme d'une émulsion de type huile dans eau ou eau dans huile, ou sous la forme d'une particule de type liposome, microsphère, nanosphère ou tout type de structure permettant l'encapsulation et la présentation sous forme particulaire de ladite fraction membranaire associée audit antigène, haptène ou complexe.

L'invention concerne aussi l'utilisation selon l'invention, caractérisée en ce que ledit agent est choisi parmi les sels d'aluminium, les sels de calcium, les composés d'origine végétale tels que le Quil A ou la saponine, ou les composés d'origine bactérienne tels que les dérivés d'anatoxine cholérique, pertussique, tétanique ou de la toxine thermolabile d'E. coli.

Est également comprise dans la présente invention, l'utilisation selon l'invention, caractérisée en ce que la composition pharmaceutique comprend en outre un agent permettant de réguler la réponse immune induite par ladite fraction membranaire associée audit antigène, haptène ou complexe.

Parmi lesdits agents de régulation, on préfère notamment les cytokines, les facteurs de croissance, les hormones ou des composants cellulaires tels que des acides nucléiques, une protéine de la famille des protéines de choc thermique ou des ribosomes.

L'invention a également pour objet l'utilisation selon l'invention, pour la préparation d'une composition pharmaceutique destinée à la prévention ou au traitement des maladies infectieuses d'origine virale, bactérienne, fongique ou parasitaire.

Parmi lesdites maladies infectieuses d'origine virale, on préfère en particulier les maladies infectieuses provoquées par les paramyxovirus, notamment par le virus parainfluenzae et de manière plus préférée par le virus respiratoire syncytial (VRS).

Dans un mode de réalisation particulier, l'utilisation selon l'invention est caractérisée en ce que ledit antigène associé à la fraction membranaire comprend le peptide G2Na, fragment de la protéine G du virus de séquence d'acides aminés SEQ ID N° 4, un peptide homologue à G2Na dont la séquence présente un taux d'identité d'au moins 80 %, de préférence 90 %, 95 % et 99 %, après alignement avec la séquence SEQ ID N° 4, ou le peptide G2Na ou l'un de ses homologues, couplé par liaison covalente à un fragment C-terminal (BB) de la protéine G de streptocoque pour former un complexe capable de se lier à la sérumalbumine de mammifère, peptide BB tel que décrit dans les documents Power et al., 1997 (Virology, 230,155-166) et WO 96/14416.

Par « pourcentage, degré ou taux d'identité » entre deux séquences d'acide nucléique ou d'acides aminés au sens de la présente invention, on entend désigner un pourcentage de nucléotides ou de résidus d'acides aminés identiques entre les deux séquences à comparer, obtenu après le meilleur alignement, ce pourcentage étant purement statistique et les différences entre les deux séquences étant réparties au hasard et sur toute leur longueur. Les comparaisons de séquences entre deux séquences d'acide nucléique ou d'acides aminés sont traditionnellement réalisées en comparant ces séquences après les avoir alignées de manière optimale, ladite comparaison étant réalisée par segment ou par « fenêtre de comparaison » pour identifier et comparer les régions locales de similarité de séquence. L'alignement optimal des séquences pour la comparaison peut être réalisé, outre manuellement, au moyen de l'algorithme d'homologie locale de Smith et Waterman (1981) [Ad. App. Math. 2:482], au moyen de l'algorithme d'homologie locale de Neddleman et Wunsch (1970) [J. Mol. Biol. 48:443], au moyen de la méthode de recherche de similarité de Pearson et Lipman (1988) [Proc. Natl. Acad. Sci. USA 85:2444], au moyen de logiciels informatiques utilisant ces algorithmes (GAP, BESTFIT, FASTA et TFASTA dans le Wisconsin Genetics Software Package, Genetics Computer Group, 575 Science Dr., Madison, WI, ou encore par les logiciels de comparaison BLAST N ou BLAST P).

Le pourcentage d'identité entre deux séquences d'acide nucléique ou d'acides aminés est déterminé en comparant ces deux séquences alignées de manière optimale par fenêtre de comparaison dans laquelle la région de la séquence d'acide nucléique ou d'acides aminés à comparer peut comprendre des additions ou des délétions par rapport à la séquence de référence pour un alignement optimal entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions dans la fenêtre de comparaison et en multipliant le résultat obtenu par 100 pour obtenir le pourcentage d'identité entre ces deux séquences.

Par exemple, on pourra utiliser le programme BLAST, « BLAST 2 sequences », disponible sur le site http://www.ncbi.nlm.nih.gov/gorf/b12.html, les paramètres utilisés étant ceux donnés par défaut (en particulier pour les paramètres « open gap penaltie » : 5, et « extension gap penaltie » : 2 ; la matrice choisie étant par exemple la matrice « BLOSUM 62 » proposée par le programme), le pourcentage d'identité entre les deux séquences à comparer étant calculé directement par le programme.

L'invention décrit ainsi un procédé de préparation d'une fraction membranaire de bactéries à gram négatif, notamment Klebsiella *pneumoniae,* caractérisé en ce qu'il comprend les étapes suivantes :
a) la culture desdites bactéries dans un milieu de culture permettant leur croissance suivie d'une centrifugation de ladite culture ;
b) le cas échéant, désactivation des enzymes lytiques du culot de bactéries obtenu à l'étape a), puis centrifugation de la suspension obtenue ;
c) extraction et élimination des protéines non membranaires et des acides nucléiques du culot obtenu à l'étape a) ou b) par au moins un cycle de lavage du culot dans une solution d'extraction ;
d) digestion du culot de membranes obtenu à l'étape c) en présence d'enzymes protéasiques suivie d'une centrifugation ;
e) au moins un cycle de lavage du culot obtenu à l'étape d) dans une solution physiologique et/ou dans de l'eau distillée ; et
f) ultrasonication du culot obtenu à l'étape e).

L'invention décrit aussi le procédé de préparation d'une fraction membranaire de bactéries à gram négatif, notamment Klebsiella *pneumoniae,* caractérisé en ce qu'il comprend les étapes suivantes :
a) la culture desdites bactéries dans un milieu de culture permettant leur croissance suivie le cas échéant d'une centrifugation ;
b) la congélation du milieu de culture ou du culot obtenu à l'étape a) suivie d'une décongélation et du séchage des cellules ;
c) élimination au moyen d'une DNase des acides nucléiques des cellules sèches obtenues à l'étape b) remises en suspension ;
d) broyage des cellules obtenues à l'étape c) et clarification de la suspension obtenue ;
e) précipitation en milieu acide de la suspension obtenue à l'étape d) et élimination du culot ;
f) neutralisation du surnageant obtenu à l'étape e) contenant la suspension membranaire, suivie d'une dialyse et d'une concentration de la suspension membranaire ; et
g) stérilisation de la suspension membranaire concentrée obtenue à l'étape f).

Le titre en protéoglycanne des fractions membranaires susceptibles d'être obtenues par lesdits procédés, principe actif de la FMKp, représenté par la somme des teneurs en galactose et en protéine, est de préférence compris :
- pour le galactose : entre 1.2 g/l et 3,4 g/l ;
- pour les protéines : entre 7,5 g/l et 14,9 g/l.

De manière plus préférée, ce titre sera :
- pour le galactose : entre 1,6 g/l et 2,6 g/l ;
- pour les protéines : entre 9,3 g/l et 11,7 g/l.

L'invention concerne en outre les compositions pharmaceutiques comprenant une fraction membranaire obtenue par les procédés ci-avant décrits selon l'utilisation de l'invention, et un antigène, haptène ou un complexe, tels que définis précédemment, mélangé à ladite fraction membranaire, ledit antigène étant choisi parmi des fragments peptidiques de paramyxovirus.

Bien entendu, lesdites compositions pharmaceutiques selon l'invention pourront comprendre en outre les agents tels que les véhicules, agents de régulation définis précédemment.

Dans un mode de réalisation préféré, la composition pharmaceutique selon l'invention est caractérisée en a que le paramyxovirus est un virus respiratoire syncytial ou un virus parainfluenzae, de préference en ce que ledit antigène associé à la fraction membranaire comprend le peptide G2Na de séquence SEQ ID N° 4 du virus respiratoire syncytial, un de ses homologues ayant au moins 80% d'identité tels que définis précédemment, ou ledit peptide G2Na, ou l'un de ses homologues ayant au moins 80% d'identité, couplé par liaison covalente à un fragment C-terminal (BB) de la protéine G de streptocoque pour former un complexe capable de se lier à la sérumalbumine de mammifère.

Les légendes des figures et exemples qui suivent sont destinés à illustrer l'invention sans aucunement en limiter la portée.
Légendes des figures :
Figure 1 : BBG2Na adjuvé par la FMKp - Etude dose-réponse (titres IgG sériques anti-G2Na).
   * p < 0,05 (par rapport au groupe PBS).
Figure 2 : BBG2Na adjuvé par la FMKp -Titres IgG1 et IgG2a anti-G2Na.
Figure 3 : BBG2Na adjuvé par la FMKp - Etude de la protection.
Figure 4 : Effet adjuvant de la FMKp vis-à-vis d'Immugrip (vaccin anti-grippal).
   * p < 0,05 par comparaison au groupe non adjuvé (« 0 ») au même jour de prélèvement.

### Exemple 1 : Obtention de la faction membranaire de K. pneumoniae (FMKp)

### Procédé N ° 1

L'extraction des membranes de K. pneumoniae I145 à partir du culot de centrifugation de l'étape est précédée de préférence par une étape de destruction des enzymes lytiques des composants cellulaires contenus dans le culot, par exemple par chauffage à 100°C de celui-ci, éventuellement après remise en solution.

L'extraction proprement dite des membranes à partir du culot de centrifugation est réalisée de préférence par traitement des composants cellulaires du culot, après une éventuelle destruction des enzymes lytiques, à l'aide d'une solution saline, par exemple du chlorure de sodium 1 M, une ou plusieurs fois, puis centrifugation, de préférence, à 20 000 g, de la suspension obtenue, le surnageant de cette centrifugation, qui est éliminé, contient les impuretés non membranaires telles que protéines et acides nucléiques, tandis que le culot contient les membranes.

Après séparation de la solution saline contenant les impuretés, les membranes sont digérées en présence d'enzymes protéolytiques, de préférence la trypsine et la chymotrypsine, en solution à pH 8 à 37°C pendant 4 heures.

Après digestion, la solution est homogénéisée par ultrasonication. Le produit ainsi obtenu constitue la fraction membranaire nommée FMKp.

Le surnageant obtenu est à nouveau centrifugé dans les mêmes conditions, de préférence à 140 000 g.

### Préparation des glycopeptides membranaires

Cette fraction est préparée à partir du culot obtenu par centrifugation à 40 000 g pendant 20 minutes. Ledit culot est remis en suspension dans du sérum physiologique puis cette suspension est portée pendant 10 minutes à 100°C dans un bain-marie d'eau bouillante pour désactiver les enzymes lytiques. Après refroidissement, on centrifuge 30 mn à 20 000 g. Le culot obtenu est extrait deux fois avec du NaCl 1M pour éliminer les protéines et les acides nucléiques. Les membranes sont recueillies par centrifugation durant 30 minutes à 20 000 g.

Elles sont ensuite soumises à une digestion par de la trypsine à pH 8 et à 37°C pendant 4 heures puis par de la chymotrypsine dans les mêmes conditions.

Les membranes sont alors recueillies par centrifugation à 2 000 g pendant 30 minutes, lavées avec du sérum physiologique puis de l'eau distillée et sont soumises à une désintégration par les ultrasons de 15 minutes.

### Procédé N° 2

Après décongélation à + 4°C pendant 48 h minimum, 1 kg de cellules sèches de K. pneumoniae est remis en suspension à 5 % cellules sèches. La DNase est ajoutée à 5 mg/l. On procède ensuite au broyage en boucle au Manton Gaulin pendant 30 min puis à une clarification sur SHARPLES à 50 1/h, suivie d'une précipitation à l'acide acétique à pH = 4,2 + 0,1 pendant 30 min. Le culot est éliminé (SHARPLES à 25 l/h) et le surnageant est neutralisé, dilué à 2 fois le volume initial avec de l'eau osmosée. Une dialyse à volume constant est alors effectuée sur PUF 100 jusqu'à 800 Ωcm, suivie d'une concentration de la suspension membranaire (SM) ainsi obtenue, à 11 l/kg de cellules sèches. On procède alors à l'autoclavage de la SM à + 121 °C durant 35 min que l'on peut conserver à + 4°C pendant 6 semaines.

### Caractéristiques de la FMKp

Par définition, le titre en protéoglycanne, principe actif de la FMKp, est égal à la somme des teneurs en galactose et en protéines.
- Galactose : en moyenne 2,2 g /1.
- Protéines : en moyenne 10,5 g/l.

### Exemple 2 : Effet adjuvant de la FMKp sur une protéine recombinante, BBG2Na

BBG2Na est une protéine recombinante produite dans E. *coli.* Elle est constituée du peptide G2Na de séquence SEQ ID N° 4, le fragment de la protéine G du Virus Respiratoire Syncytial (VRS) de type A allant du résidu 130 au résidu 230, fusionnée à BB, un fragment de la protéine G des Streptocoques, ayant la capacité de se lier à l'albumine sérique. BBG2Na est un vaccin anti-VRS (Power U. Virology 1997, 230:155-166).

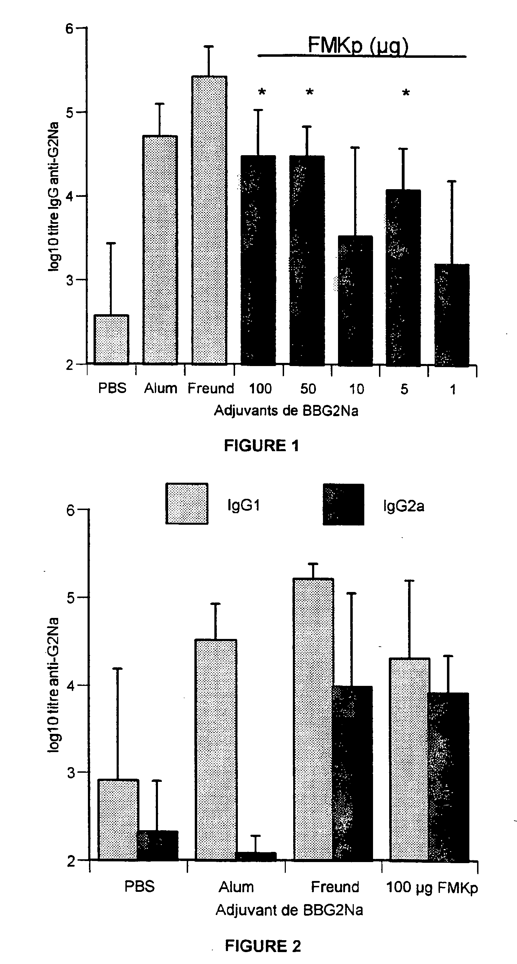

Des souris BALB/c reçoivent 2 injections par voie sous-cutanée de 20 µg de BBG2Na et différentes quantités de FMKp. Des prélèvements de sang sont effectués à J28 et les titres anticorps sériques sont déterminés par ELISA avec G2Na en phase solide. Les résultats obtenus sont illustrés par la figure 1. De manière surprenante, ils montrent que la FMKp augmente significativement la réponse IgG anti-G2Na ; le titre IgG anti-G2Na atteint est similaire à ceux induits par l'alum ou l'adjuvant de Freund. L'effet est dose-dépendant : il est observé à partir de 5 µg de FMKp, maximal à partir de 50 µg de FMKp et reste stable avec 100 µg de FMKp. La FMKp est donc un adjuvant potentiel pour BBG2Na.

Afin de connaître l'effet de la FMKp sur l'orientation de la réponse immunitaire, en terme de réponse Th1/Th2, les titres IgG1 et IgG2a anti-G2Na ont été déterminés sur des sérums obtenus comme précisé ci-dessus. Les résultats (figure 2) montrent qu'étonnamment, la FMKp est capable de modifier le ratio IgGl/IgG2a anti-G2Na, à l'inverse de ce qui est observé avec l'alum, pour lequel l'isotype majoritaire est l'IgG1. Ce profil est proche de celui induit par l'adjuvant de Freund. Ceci indique que la FMKp peut être utilisée comme adjuvant de l'immunité pour induire une réponse de type mixte (Th1/Th2).

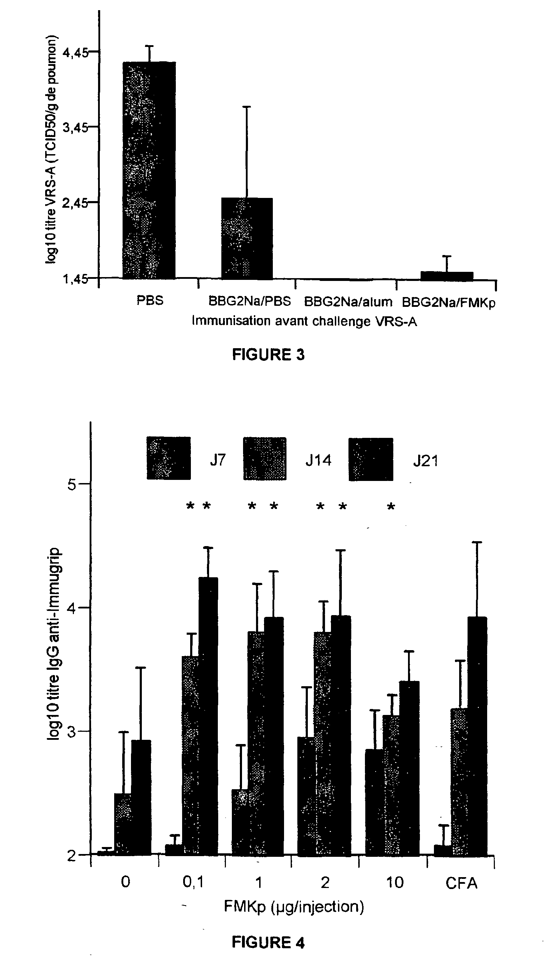

Les animaux immunisés comme décrit ci-dessus ont reçu une épreuve virale par voie nasale avec 10⁵ TCID₅₀ de VRS-A. Celle-ci a été effectuée 3 semaines après la dernière immunisation. Cinq jours après l'épreuve virale, les animaux ont été sacrifiés, et les poumons prélevés afin de déterminer les titres de VRS-A. Les résultats (figure 3) montrent que les animaux ayant reçu BBG2Na adjuvé par la FMKp sont protégés contre un challenge à RSV-A.

En conclusion, la FMKp permet de réorienter la réponse anticorps sans affecter la capacité de protection des souris contre un challenge à VRS-A.

### Exemple 3 : Effet adjuvant de la FMKp sur un virus inactivé (vaccin anti-grippal)

Des souris BALB/c reçoivent 1 seule injection de 0,01 µg d'Immugrip™ (vaccin anti-grippal commercialisé par les Laboratoires INAVA), et de différentes quantités de FMKp. Les produits sont coadministrés. L'injection se fait par voie sous-cutanée à J0. Des prélèvements sanguins sont effectués à J7, J14 et J21. Le titre anticorps IgG sériques anti-Immugrip se fait par ELISA avec Immugrip à 2 µg/ml en phase solide. Les résultats présentés (figure 4) montrent que la FMKp augmente significativement le titre anticorps anti-Immugrip et ceci dès la plus faible dose de FMKp, à savoir 0,1 µg. L'effet adjuvant est dose-dépendant. On remarque de manière intéressante, que la présence de la FMKp induit la génération d'une réponse anticorps plus précoce, observée dès J7, comparée au contrôle Immugrip non adjuvé. Cet effet n'est pas obtenu avec l'adjuvant de référence, l'adjuvant complet de Freund (CFA).

## Revendications

1. Utilisation d'une fraction membranaire de Klebsiella *pneumoniae* mélangée à un antigène ou haptène, ledit antigène ou haptène est choisi parmi les antigènes ou haptènes spécifiques d'un agent infectieux ou parmi les antigènes associés à des cellules tumorales et ledit mélange étant capable d'orienter la réponse immunitaire vers une réponse de type Th1 et/ou de type mixte Th1/Th2 dirigée contre ledit antigène ou haptène, réponse dans laquelle la réponse Th1 est voisine ou supérieure à la réponse de type Th2, pour la préparation d'une composition pharmaceutique destinée à la prévention ou au traitement des maladies infectieuses ou des cancers, **caractérisée en ce que** la fraction membranaire est préparée par un procédé comprenant les étapes suivantes :
a) la culture desdites bactéries dans un milieu de culture permettant leur croissance suivie d'une centrifugation de ladite culture ;
b) le cas échéant, désactivation des enzymes lytiques du culot de bactéries obtenu à l'étape a), puis centrifugation de la suspension obtenue ;
c) extraction et élimination des protéines non membranaires et des acides nucléiques du culot obtenu à l'étape a) ou b) par au moins un cycle de lavage du culot dans une solution d'extraction ;
d) digestion du culot de membranes obtenu à l'étape c) en présence d'enzymes protéasiques suivie d'une centrifugation ;
e) au moins un cycle de lavage du culot obtenu à l'étape d) dans une solution physiologique et/ou dans de l'eau distillée ; et
f) ultrasonication du culot obtenu à l'étape e), ou
par un procédé comprenant les étapes suivantes :
a) la culture desdites bactéries dans un milieu de culture permettant leur croissance suivie le cas échéant d'une centrifugation ;
b) la congélation du milieu de culture ou du culot obtenu à l'étape a) suivie d'une décongélation et du séchage des cellules ;
c) élimination au moyen d'une DNase des acides nucléiques des cellules sèches obtenues à l'étape b) remises en suspension ;
d) broyage des cellules obtenues à l'étape c) et clarification de la suspension obtenue ;
e) précipitation en milieu acide de la suspension obtenue à l'étape d) et élimination du culot ;
f) neutralisation du surnageant obtenu à l'étape e) contenant la suspension membranaire, suivie d'une dialyse et d'une concentration de la suspension membranaire ; et
g) stérilisation de la suspension membranaire concentrée obtenue à l'étape f).

2. Utilisation selon la revendication 1, **caractérisée en ce que** la fraction membranaire comprend au moins des fractions membranaires de deux souches différentes de bactéries.

3. Utilisation selon la revendication 2, **caractérisée en ce que** ledit antigène ou haptène est choisi parmi les peptides, les lipopeptides, les polysaccharides, les oligosaccharides, les acides nucléiques ou les lipides.

4. Utilisation selon l'une des revendications 1 à 3, **caractérisée en ce que** ledit antigène ou haptène est couplé de manière covalente à un peptide support pour former un complexe capable de se lier spécifiquement à la sérumalbumine de mammifère.

5. Utilisation selon la revendication 4, **caractérisée en ce que** ledit peptide support est un fragment peptidique issu de la protéine G du streptocoque.

6. Utilisation selon l'une des revendications 4 et 5, **caractérisée en ce que** ledit complexe est préparé par recombinaison génétique.

7. Utilisation selon l'une des revendications 1 à 6, **caractérisée en ce que** la composition pharmaceutique comprend en outre un agent permettant de véhiculer ladite fraction membranaire associée audit antigène, haptène ou complexe sous une forme permettant d'améliorer sa stabilité et/ou son immunogénicité.

8. Utilisation selon la revendication 7, **caractérisée en ce que** ledit agent est une émulsion de type huile dans eau ou eau dans huile.

9. Utilisation selon la revendication 7, **caractérisée en ce que** ledit agent est une particule de type liposome, microsphère, nanosphère ou tout type de structure permettant l'encapsulation et la présentation sous forme particulaire de ladite fraction membranaire associée audit antigène, haptène ou complexe.

10. Utilisation selon la revendication 7, **caractérisée en ce que** ledit agent est choisi parmi les sels d'aluminium, les sels de calcium, les composés d'origine végétale tels que le Quil A ou la saponine, ou les composés d'origine bactérienne tels que les dérivés d'anatoxine cholérique, pertussique, tétanique ou de la toxine thermolabile d'E. coli.

11. Utilisation selon l'une des revendications 1 à 10, **caractérisée en ce que** la composition pharmaceutique comprend en outre un agent permettant de réguler la réponse immune induite par ladite fraction membranaire associée audit antigène, haptène ou complexe.

12. Utilisation selon la revendication 11, **caractérisée en ce que** ledit agent de régulation est choisi parmi les cytokines, les facteurs de croissance, les hormones ou des composants cellulaires tels que des acides nucléiques, une protéine de la famille des protéines de choc thermique ou des ribosomes.

13. Utilisation selon l'une des revendications 1 à 12, pour la préparation d'une composition pharmaceutique destinée à la prévention ou au traitement des maladies infectieuses, **caractérisée en ce que** la maladie infectieuse est d'origine virale, bactérienne, fongique ou parasitaire.

14. Utilisation selon la revendication 13, pour la préparation d'une composition pharmaceutique destinée à la prévention ou au traitement des infections par les paramyxovirus.

15. Utilisation selon la revendication 14, **caractérisée en ce que** le paramyxovirus est un virus respiratoire syncytial.

16. Utilisation selon la revendication 15, **caractérisée en ce que** ledit antigène associé à la fraction membranaire comprend le peptide G2Na de séquence SEQ ID N° 4 ou l'un de ses homologues dont la séquence présente un degré d'identité d'au moins 80 % avec la séquence SEQ ID N° 4.

17. Utilisation selon la revendication 16, **caractérisée en ce que** ledit peptide G2Na ou l'un de ses homologues est couplé par liaison covalente à un fragment C-terminal (BB) de la protéine G de streptocoque pour former un complexe capable de se lier à la sérumalbumine de mammifère.

18. Utilisation selon la revendication 14, **caractérisée en ce que** le paramyxovirus est un virus parainfluenzae.

19. Composition pharmaceutique, **caractérisée en ce qu'**elle comprend une fraction membranaire et un antigène ou haptène mélangé à ladite fraction membranaire,
**caractérisée en ce que** ledit antigène est choisi parmi des fragments peptidiques de paramyxovirus et **caractérisée en ce que** la fraction membranaire est préparée par un procédé comprenant les étapes suivantes :
a) la culture desdites bactéries dans un milieu de culture permettant leur croissance suivie d'une centrifugation de ladite culture ;
b) le cas échéant, désactivation des enzymes lytiques du culot de bactéries obtenu à l'étape a), puis centrifugation de la suspension obtenue ;
c) extraction et élimination des protéines non membranaires et des acides nucléiques du culot obtenu à l'étape a) ou b) par au moins un cycle de lavage du culot dans une solution d'extraction ;
d) digestion du culot de membranes obtenu à l'étape c) en présence d'enzymes protéasiques suivie d'une centrifugation ;
e) au moins un cycle de lavage du culot obtenu à l'étape d) dans une solution physiologique et/ou dans de l'eau distillée ; et
f) ultrasonication du culot obtenu à l'étape e), ou
par un procédé comprenant les étapes suivantes :
a) la culture desdites bactéries dans un milieu de culture permettant leur croissance suivie le cas échéant d'une centrifugation ;
b) là congélation du milieu de culture ou du culot obtenu à l'étape a) suivie d'une décongélation et du séchage des cellules ;
c) élimination au moyen d'une DNase des acides nucléiques des cellules sèches obtenues à l'étape b) remises en suspension ;
d) broyage des cellules obtenues à l'étape c) et clarification de la suspension obtenue ;
e) précipitation en milieu acide de la suspension obtenue à l'étape d) et élimination du culot ;
f) neutralisation du surnageant obtenu à l'étape e) contenant la suspension membranaire, suivie d'une dialyse et d'une concentration de la suspension membranaire ; et
g) stérilisation de la suspension membranaire concentrée obtenue à l'étape f).

20. Composition pharmaceutique selon la revendication 19, **caractérisée en ce que** le paramyxovirus est un virus respiratoire syncytial ou un virus parainfluenzae.

21. Composition pharmaceutique selon la revendication 20, **caractérisée en ce que** ledit antigène associé à la fraction membranaire comprend le peptide G2Na de séquence SEQ ID N° 4 du virus respiratoire syncytial, ou un peptide dont la séquence présente un degré d'identité d'au moins 80 % avec la séquence SEQ ID N° 4.

22. Composition pharmaceutique selon la revendication 20, **caractérisée en ce que** ledit peptide G2Na ou l'un de ses homologues est couplé par liaison covalente à un fragment C-terminal (BB) de la protéine G de streptocoque pour former un complexe capable de se lier à la sérumalbumine de mammifère.

## Claims

1. Use of a Klebsiella *pneumoniae* membrane fraction mixed with an antigen or hapten, said antigen or hapten being chosen from the antigens or haptens specific to an infectious agent or from the antigens associated with tumour cells and said mixture being capable of orienting the immune response towards a Th1 type and/or mixed Thl/Th2 type response directed against said antigen or hapten, a response in which the Th1 response is close to or greater than the Th2 type response, for the preparation of a pharmaceutical composition intended to prevent or to treat infectious diseases or cancers, **characterized in that** the membrane fraction is prepared by a method comprising the following steps:
a) culture of said bacteria in a culture medium allowing their growth followed by centrifugation of said culture;
b) where appropriate, deactivation of the lytic enzymes of the bacterial pellet obtained in step a), followed by centrifugation of the suspension obtained;
c) extraction and removal of non-membrane proteins and of nucleic acids from the pellet obtained in step a) or b) by at least one cycle of washing the pellet in an extraction solution;
d) digestion of the membrane pellet obtained in step c) in the presence of protease enzymes, followed by centrifugation;
e) at least one cycle of washing of the pellet obtained in step d) in physiological saline and/or in distilled water; and
f) ultrasonication of the pellet obtained in step e), or
by a method comprising the following steps:
a) culture of said bacteria in a culture medium allowing their growth, followed, where appropriate, by centrifugation;
b) freezing of the culture medium or of the pellet obtained in step a) followed by thawing and drying of the cells;
c) removal, by means of a DNase, of the nucleic acids from the dry cells obtained in step b) which have been resuspended;
d) grinding of the cells obtained in step c) and clarification of the suspension obtained;
e) precipitation, in an acid medium, of the suspension obtained in step d) and removal of the pellet;
f) neutralization of the supernatant obtained in step e) containing the membrane suspension, followed by dialysis and concentration of the membrane suspension; and
g) sterilization of the concentrated membrane suspension obtained in step f).

2. Use according to Claim 1, **characterized in that** the membrane fraction comprises at least membrane fractions of two different bacterial strains.

3. Use according to Claim 2, **characterized in that** said antigen or hapten is chosen from peptides, lipopeptides, polysaccharides, oligosaccharides, nucleic acids or lipids.

4. Use according to one of Claims 1 to 3, **characterized in that** said antigen or hapten is covalently coupled with a supporting peptide to form a complex capable of specifically binding to mammalian serum albumin.

5. Use according to Claim 4, **characterized in that** said supporting peptide is a peptide fragment derived from streptococcal G protein.

6. Use according to either of Claims 4 and 5, **characterized in that** said complex is prepared by genetic recombination.

7. Use according to one of Claims 1 to 6, **characterized in that** the pharmaceutical composition comprises, in addition, an agent which makes it possible to carry said membrane fraction associated with said antigen, hapten or complex in a form which makes it possible to enhance its stability and/or its immunogenicity.

8. Use according to Claim 7, **characterized in that** said agent is an oil-in-water or water-in-oil type emulsion.

9. Use according to Claim 7, **characterized in that** said agent is a particle of the liposome, microsphere or nanosphere type or any type of structure allowing the encapsulation and the presentation in particulate form of said membrane fraction associated with said antigen, hapten or complex.

10. Use according to Claim 7, **characterized in that** said agent is chosen from aluminium salts, calcium salts, compounds of plant origin such as Quil A or saponin, or compounds of bacterial origin such as cholera, pertussis or tetanus toxoid or thermolabile E. coli toxin.

11. Use according to one of Claims 1 to 10, **characterized in that** the pharmaceutical composition comprises, in addition, an agent which makes it possible to regulate the immune response induced by said membrane fraction associated with said antigen, hapten or complex.

12. Use according to Claim 11, **characterized in that** said regulatory agent is chosen from cytokines, growth factors, hormones or cellular components such as nucleic acids, a protein of the family of heat shock proteins or ribosomes.

13. Use according to one of Claims 1 to 12, for the preparation of a pharmaceutical composition intended for the prevention or treatment of infectious diseases, **characterized in that** the infectious disease is of viral, bacterial, fungal or parasitic origin.

14. Use according to Claim 13, for the preparation of a pharmaceutical composition intended for the prevention or treatment of paramyxovirus infections.

15. Use according to Claim 14, **characterized in that** the paramyxovirus is a respiratory syncytial virus.

16. Use according to Claim 15, **characterized in that** said antigen associated with the membrane fraction comprises the peptide G2Na having the sequence SEQ ID No. 4 or one of its homologues whose sequence exhibits a degree of identity of at least 80% with the sequence SEQ ID No. 4.

17. Use according to Claim 16, **characterized in that** said peptide G2Na or one of its homologues is covalently coupled with a C-terminal fragment (BB) of the streptococcal G protein to form a complex capable of binding to mammalian serum albumin.

18. Use according to Claim 14, **characterized in that** the paramyxovirus is a parainfluenzae virus.

19. Pharmaceutical composition, **characterized in that** it comprises a membrane fraction and an antigen or hapten mixed with said membrane fraction, **characterized in that** said antigen is chosen from paramyxovirus peptide fragments and **characterized in that** the membrane fraction is prepared by a method comprising the following steps:
a) culture of said bacteria in a culture medium allowing their growth followed by centrifugation of said culture;
b) where appropriate, deactivation of the lytic enzymes of the bacterial pellet obtained in step a), followed by centrifugation of the suspension obtained;
c) extraction and removal of non-membrane proteins and of nucleic acids from the pellet obtained in step a) or b) by at least one cycle of washing the pellet in an extraction solution;
d) digestion of the membrane pellet obtained in step c) in the presence of protease enzymes, followed by centrifugation;
e) at least one cycle of washing of the pellet obtained in step d) in physiological saline and/or in distilled water; and
f) ultrasonication of the pellet obtained in step e), or
by a method comprising the following steps:
a) culture of said bacteria in a culture medium allowing their growth, followed, where appropriate, by centrifugation;
b) freezing of the culture medium or of the pellet obtained in step a) followed by thawing and drying of the cells;
c) removal, by means of a DNase, of the nucleic acids from the dry cells obtained in step b) which have been resuspended;
d) grinding of the cells obtained in step c) and clarification of the suspension obtained;
e) precipitation, in an acid medium, of the suspension obtained in step d) and removal of the pellet;
f) neutralization of the supernatant obtained in step e) containing the membrane suspension, followed by dialysis and concentration of the membrane suspension; and
g) sterilization of the concentrated membrane suspension obtained in step f).

20. Pharmaceutical composition according to Claim 19, **characterized in that** the paramyxovirus is a respiratory syncytial virus or a parainfluenzae virus.

21. Pharmaceutical composition according to Claim 20, **characterized in that** said antigen associated with the membrane fraction comprises the peptide G2Na having the sequence SEQ ID No. 4 of the respiratory syncytial virus or a peptide whose sequence exhibits a degree of identity of at least 80% with the sequence SEQ ID No. 4.

22. Pharmaceutical composition according to Claim 20, **characterized in that** said peptide G2Na or one of its homologues is covalently coupled with a C-terminal fragment (BB) of the streptococcal G protein to form a complex capable of binding to mammalian serum albumin.

## Patentansprüche

1. Verwendung einer Membranfraktion von Klebsiella *pneumoniae,* welche mit einem Antigen oder Hapten gemischt ist, wobei das Antigen oder Hapten unter den für ein infektiöses Agens spezifischen Antigenen oder Haptenen oder unter den mit Tumorzellen assoziierten Antigenen ausgewählt wird und wobei die Mischung in der Lage ist, die Immunantwort in Richtung einer Antwort vom Th1-Typ und/oder vom gemischten Th1/Th2-Typ, welche gegen das Antigen oder Hapten gerichtet ist, zu dirigieren, bei welcher Antwort die Th1-Antwort der Antwort vom Th2-Typ nahe kommt oder stärker als jene ist, für die Herstellung einer pharmazeutischen Zusammensetzung, welche für die Verhütung oder die Behandlung von Infektionskrankheiten oder Krebserkrankungen bestimmt ist, **dadurch gekennzeichnet, dass** die Membranfraktion hergestellt wird durch ein Verfahren, welches die folgenden Schritte umfasst:
a) die Kultivierung der Bakterien in einem Kulturmedium, welches deren Vermehrung erlaubt, gefolgt von einer Zentrifugation der Kultur;
b) gegebenenfalls Inaktivierung der lytischen Enzyme des Bodensatzes von Bakterien, der in Schritt a) erhalten wird, dann Zentrifugation der erhaltenen Suspension;
c) Extraktion und Entfernung der Nicht-Membranproteine und der Nukleinsäuren aus dem in Schritt a) oder b) erhaltenen Bodensatz durch wenigstens einen Waschzyklus des Bodensatzes in einer Extraktionslösung;
d) Verdau des Bodensatzes von Membranen, der in Schritt c) erhalten wird, in Gegenwart von Protease-Enzymen, gefolgt von einer Zentrifugation;
e) wenigstens einen Waschzyklus des in Schritt d) erhaltenen Bodensatzes in einer physiologischen Lösung und/oder in destilliertem Wasser; und
f) Ultraschallbehandlung des in Schritt e) erhaltenen Bodensatzes; oder
durch ein Verfahren, welches die folgenden Schritte umfasst:
a) die Kultivierung der Bakterien in einem Kulturmedium, welches deren Vermehrung erlaubt, gegebenenfalls gefolgt von einer Zentrifugation;
b) das Einfrieren des Kulturmediums oder des Bodensatzes, welches bzw. welcher in Schritt a) erhalten wird, gefolgt von einem Auftauen und der Trocknung der Zellen;
c) Entfernung der Nukleinsäuren der in Schritt b) erhaltenen trockenen Zellen, die resuspendiert worden sind, mittels einer DNase;
d) Aufbrechen der in Schritt c) erhaltenen Zellen und Klärung der erhaltenen Suspension;
e) Ausfällung der in Schritt d) erhaltenen Suspension in saurem Milieu und Entfernung des Bodensatzes;
f) Neutralisation des in Schritt e) erhaltenen Überstands, welcher die Membransuspension enthält, gefolgt von einer Dialyse und einer Aufkonzentrierung der Membransuspension; und
g) Sterilisation der in Schritt f) erhaltenen aufkonzentrierten Membransuspension.

2. Verwendung nach Anspruch 1, **dadurch gekennzeichnet, dass** die Membranfraktion wenigstens Membranfraktionen von zwei unterschiedlichen Bakterienstämmen umfasst.

3. Verwendung nach Anspruch 2, **dadurch gekennzeichnet, dass** das Antigen oder Hapten unter den Peptiden, den Lipopeptiden, den Polysacchariden, den Oligosacchariden, den Nukleinsäuren oder den Lipiden ausgewählt wird.

4. Verwendung nach einem der Ansprüche 1 bis 3, **dadurch gekennzeichnet, dass** das Antigen oder Hapten kovalent an ein Trägerpeptid gebunden ist, um einen Komplex zu bilden, welcher in der Lage ist, sich spezifisch an Säugetier-Serumalbumin zu binden.

5. Verwendung nach Anspruch 4, **dadurch gekennzeichnet, dass** das Trägerpeptid ein Peptidfragment ist, welches von dem Protein G von Streptokokken stammt.

6. Verwendung nach einem der Ansprüche 4 und 5, **dadurch gekennzeichnet, dass** der Komplex durch genetische Rekombination hergestellt wird.

7. Verwendung nach einem der Ansprüche 1 bis 6, **dadurch gekennzeichnet, dass** die pharmazeutische Zusammensetzung außerdem ein Mittel umfasst, welches erlaubt, die mit dem Antigen, Hapten oder Komplex assoziierte Membranfraktion in einer Form mitzuführen, welche erlaubt, deren Stabilität und/oder deren Immunogenität zu verbessern.

8. Verwendung nach Anspruch 7, **dadurch gekennzeichnet, dass** das Mittel eine Emulsion vom ÖI-in-Wasser- oder Wasser-in-Öl-Typ ist.

9. Verwendung nach Anspruch 7, **dadurch gekennzeichnet, dass** das Mittel ein Teilchen vom Typ Liposom, Mikrosphäre, Nanosphäre oder eine jegliche Art von Struktur, welche die Verkapselung und die Präsentation der mit dem Antigen, Hapten oder Komplex assoziierten Membranfraktion in partikulärer Form erlaubt, ist.

10. Verwendung nach Anspruch 7, **dadurch gekennzeichnet, dass** das Mittel unter den Aluminiumsalzen, den Calciumsalzen, den Verbindungen pflanzlicher Herkunft, wie Quil A oder Saponin, oder den Verbindungen bakterieller Herkunft, wie den Derivaten von Cholera-Anatoxin, Pertussis-Anatoxin, Tetanustoxoid oder dem thermolabilen Toxin von E. coli, ausgewählt wird.

11. Verwendung nach einem der Ansprüche 1 bis 10, **dadurch gekennzeichnet, dass** die pharmazeutische Zusammensetzung außerdem ein Mittel umfasst, welches erlaubt, die durch die mit dem Antigen, Hapten oder Komplex assoziierte Membranfraktion induzierte lmmunantwort zu regulieren.

12. Verwendung nach Anspruch 11, **dadurch gekennzeichnet, dass** das Regulationsmittel unter den Zytokinen, den Wachstumsfaktoren, den Hormonen oder Zellbestandteilen, wie Nukleinsäuren, einem Protein aus der Familie der Hitzeschockproteine oder Ribosomen, ausgewählt wird.

13. Verwendung nach einem der Ansprüche 1 bis 12 für die Herstellung einer pharmazeutischen Zusammensetzung, die für die Verhütung oder Behandlung von Infektionskrankheiten bestimmt ist, **dadurch gekennzeichnet, dass** die Infektionskrankheit viralen, bakteriellen, pilzlichen oder parasitären Ursprungs ist.

14. Verwendung nach Anspruch 13 für die Herstellung einer pharmazeutischen Zusammensetzung, die für die Verhütung oder Behandlung von Infektionen durch Paramyxoviren bestimmt ist.

15. Verwendung nach Anspruch 14, **dadurch gekennzeichnet, dass** das Paramyxovirus ein Respiratory-Syncytial-Virus ist.

16. Verwendung nach Anspruch 15, **dadurch gekennzeichnet, dass** das mit der Membranfraktion assoziierte Antigen das Peptid G2Na mit der Sequenz SEQ ID Nr. 4 oder eines von dessen Homologen, dessen Sequenz ein Identitätsausmaß von wenigstens 80% zu der Sequenz SEQ ID Nr. 4 aufweist, umfasst.

17. Verwendung nach Anspruch 16, **dadurch gekennzeichnet, dass** das Peptid G2Na oder das eine von dessen Homologen durch kovalente Bindung mit einem C-terminalen Fragment (BB) des Proteins G von Streptokokken verknüpft ist, um einen Komplex zu bilden, der in der Lage ist, sich an Säugetier-Serumalbumin zu binden.

18. Verwendung nach Anspruch 14, **dadurch gekennzeichnet, dass** das Paramyxovirus ein Parainfluenzavirus ist.

19. Pharmazeutische Zusammensetzung, **dadurch gekennzeichnet, dass** sie eine Membranfraktion und ein Antigen oder Hapten, das mit der Membranfraktion gemischt ist, umfasst, **dadurch gekennzeichnet, dass** das Antigen unter Peptidfragmenten von Paramyxoviren ausgewählt wird, und **dadurch gekennzeichnet, dass** die Membranfraktion hergestellt wird durch ein Verfahren, welches die folgenden Schritte umfasst:
a) die Kultivierung der Bakterien in einem Kulturmedium, welches deren Vermehrung erlaubt, gefolgt von einer Zentrifugation der Kultur;
b) gegebenenfalls Inaktivierung der lytischen Enzyme des Bodensatzes von Bakterien, der in Schritt a) erhalten wird, dann Zentrifugation der erhaltenen Suspension;
c) Extraktion und Entfernung der Nicht-Membranproteine und der Nukleinsäuren aus dem in Schritt a) oder b) erhaltenen Bodensatz durch wenigstens einen Waschzyklus des Bodensatzes in einer Extraktionslösung;
d) Verdau des Bodensatzes von Membranen, der in Schritt c) erhalten wird, in Gegenwart von Protease-Enzymen, gefolgt von einer Zentrifugation;
e) wenigstens einen Waschzyklus des in Schritt d) erhaltenen Bodensatzes in einer physiologischen Lösung und/oder in destilliertem Wasser; und
f) Ultraschallbehandlung des in Schritt e) erhaltenen Bodensatzes; oder
durch ein Verfahren, welches die folgenden Schritte umfasst:
a) die Kultivierung der Bakterien in einem Kulturmedium, welches deren Vermehrung erlaubt, gegebenenfalls gefolgt von einer Zentrifugation;
b) das Einfrieren des Kulturmediums oder des Bodensatzes, welches bzw. welcher in Schritt a) erhalten wird, gefolgt von einem Auftauen und der Trocknung der Zellen;
c) Entfernung der Nukleinsäuren der in Schritt b) erhaltenen trockenen Zellen, die resuspendiert worden sind, mittels einer DNase;
d) Aufbrechen der in Schritt c) erhaltenen Zellen und Klärung der erhaltenen Suspension;
e) Ausfällung der in Schritt d) erhaltenen Suspension in saurem Milieu und Entfernung des Bodensatzes;
f) Neutralisation des in Schritt e) erhaltenen Überstands, welcher die Membransuspension enthält, gefolgt von einer Dialyse und einer Aufkonzentrierung der Membransuspension; und
g) Sterilisation der in Schritt f) erhaltenen aufkonzentrierten Membransuspension.

20. Pharmazeutische Zusammensetzung nach Anspruch 19, **dadurch gekennzeichnet, dass** das Paramyxovirus ein Respiratory-Syncytial-Virus oder ein Parainfluenzavirus ist.

21. Pharmazeutische Zusammensetzung nach Anspruch 20, **dadurch gekennzeichnet, dass** das mit der Membranfraktion assoziierte Antigen das Peptid G2Na mit der Sequenz SEQ ID Nr. 4 des Respiratory-Syncytial-Virus oder ein Peptid, dessen Sequenz ein ldentitätsausmaß von wenigstens 80% zu der Sequenz SEQ ID Nr. 4 aufweist, umfasst.

22. Pharmazeutische Zusammensetzung nach Anspruch 20, **dadurch gekennzeichnet, dass** das Peptid G2Na oder das eine von dessen Homologen durch kovalente Bindung mit einem C-terminalen Fragment (BB) des Proteins G von Streptokokken verknüpft ist, um einen Komplex zu bilden, der in der Lage ist, sich an Säugetier-Serumalbumin zu binden.
